# EUROPÄISCHE PATENTSCHRIFT

(11) **EP 2 593 891 B1**
(45) Veröffentlichungstag und Bekanntmachung des Hinweises auf die Patenterteilung: **30.10.2019**
(21) Anmeldenummer: 11787591.4
(22) Anmeldetag: 12.07.2011
(51) Int. Cl.: G16H 10/60, G16H 50/70

(54) **VERFAHREN ZUR ÜBERWACHUNG DER MEDIZINISCHEN PARAMETER EINES PATIENTEN**
METHOD FOR MONITORING THE MEDICAL PARAMETERS OF A PATIENT
PROCÉDÉ DE SURVEILLANCE DES PARAMÈTRES MÉDICAUX D'UN PATIENT

(30) Priorität: 16.07.2010 DE 102010027486
(43) Veröffentlichungstag der Anmeldung: 22.05.2013
(73) Patentinhaber: Meierhofer Medizintechnik GmbH, 04107 Leipzig (DE)
(72) Erfinder: LÖSER, Thomas, 04275 Leipzig (DE)
(74) Vertreter: Carlsohn, Alexander
(86) Internationale Anmeldenummer: PCT/DE2011/075162
(87) Internationale Veröffentlichungsnummer: WO 2012/022335

(56) Entgegenhaltungen:
- EP-A2- 1 640 888
- WO-A1-2009/043144
- WO-A2-2010/009717
- US-A1- 2005 027 182
- US-A1- 2008 249 386
- "STATEMENT IN ACCORDANCE WITH THE NOTICE FROM THE EUROPEAN PATENT OFFICE DATED 1 OCTOBER 2007 CONCERNING BUSINESS METHODS - PCT / ERKLAERUNG GEMAESS DER MITTEILUNG DES EUROPAEISCHEN PATENTAMTS VOM 1.OKTOBER 2007 UEBER GESCHAEFTSMETHODEN - PCT / DECLARATION CONFORMEMENT AU COMMUNIQUE DE L'OFFICE EUROP", 20071101, 1. November 2007 (2007-11-01), XP002456414,
- EPO: "Mitteilung des Europäischen Patentamts vom 1. Oktober 2007 über Geschäftsmethoden = Notice from the European Patent Office dated 1 October 2007 concerning business methods = Communiqué de l'Office européen des brevets,en date du 1er octobre 2007, concernant les méthodes dans le domaine des activités", JOURNAL OFFICIEL DE L'OFFICE EUROPEEN DES BREVETS.OFFICIAL JOURNAL OF THE EUROPEAN PATENT OFFICE.AMTSBLATTT DES EUROPAEISCHEN PATENTAMTS, OEB, MUNCHEN, DE, Bd. 30, Nr. 11, 1. November 2007 (2007-11-01), Seiten 592-593, XP002498048, ISSN: 0170-9291

## Beschreibung

Die Erfindung betrifft ein Verfahren zur Überwachung der medizinischen Parameter eines Patienten.

Im Gesundheitswesen, insbesondere in Kliniken, werden Patientendaten-Managementsysteme eingesetzt, in denen Patientendaten gespeichert werden. Die Patientendaten umfassen neben den sogenannten Stammdaten des Patienten wie Name, Vorname und Geburtsdatum die medizinischen Befunde und eingeleiteten oder bereits abgeschlossenen ärztlichen oder pflegerischen Maßnahmen. Derartige Maßnahmen werden im Folgenden auch als medizinische Maßnahmen bezeichnet.

Bekannt sind ferner Systeme, die einen Arzt bei der Entscheidung, welche medizinische Maßnahme in einer gegebenen Situation die richtige ist, unterstützen. Beispielsweise offenbart WO 2008/044189 A2 ein Entscheidungsunterstützungssystem, das bei der Behandlung einer Sepsis eingesetzt werden soll.

Eine Sepsis ist definiert als "die Gesamtheit der lebensbedrohlichen klinischen Krankheitserscheinungen und pathophysiologischen Veränderungen als Reaktion auf die Aktion pathogener Keime und ihrer Produkte, die aus einem Infektionsherd in den Blutstrom eindringen, die großen biologischen Kaskadensysteme und spezielle Zellsysteme aktivieren und die Bildung und Freisetzung humoraler und zellulärer Mediatoren auslösen" (Schuster, H. P., und Müller-Werdan, U.: Definition und Diagnose von Sepsis und Multiorganversagen in Sepsis und MODS, Berlin 2005). Ist zusätzlich ein Organ des Patienten akut geschädigt, liegt eine schwere Sepsis vor.

Typischer Erreger einer Sepsis sind *Staphylococcus aureus,* koagulase-negative Staphylokokken, Enterkokken, *Escheria coli, Klebsiella spp., Enterbacter ssp.* und *Pseudomonas aeruginosa.*

Am Beginn einer intensivmedizinischen Behandlung, d. h. in der Regel bei der Einlieferung eines Patienten in die Intensivstation eines Krankenhauses, ist häufig nicht bekannt, ob der Patient an einer Infektion leidet oder nicht. Bekannt ist hingegen, welche Pathogene, insbesondere welche Keime, auf der Intensivstation bereits vorhanden sind. Aus diesem Grunde werden im Rahmen einer sogenannten "kalkulierten Therapie" sofort mit Beginn der intensivmedizinischen Behandlung ein oder mehrere Medikamente gegeben, mit denen typische Infektionen, insbesondere die Infektionen, die durch die Keime hervorgerufen werden, die auf der Station bekanntlich vorhanden sind, bekämpft werden sollen. Derartige Medikamente umfassen z. B. Antibiotika, Virostatika und Antimykotika, wobei die kalkulierte Therapie typischerweise die Gabe eines Antibiotikums vorsieht.

Gleichzeitig wird dem Patienten am Beginn der intensivmedizinischen Behandlung eine Probe entnommen, die auf pathogene Keime untersucht wird. Die Untersuchung, die beispielsweise mit dem Nachweis von Antikörpern oder der Erbinformation von Erregern (z. B. Specis-PCR) oder dem Anzüchten von Zellkulturen verbunden ist, wird in spezialisierten Labors durchgeführt. Die Ergebnisse dieser Untersuchung liegen typischerweise nach drei Tagen vor. Die Ergebnisse lassen erkennen, welche pathogenen Keime und welche Resistenzen bei dem Patienten vorliegen. Sobald dies bekannt ist, wird die medizinische Behandlung an den tatsächlichen Zustand des Patienten angepasst.

Die in den Patientendaten-Managementsystemen gespeicherten Daten werden durch die den Patienten behandelnden Personen sowie durch das Laborpersonal in das System über standardisierte Schnittstellen (in Krankenhäusern HL7, in Praxen xDT) eingegeben und auf Anforderung dem Behandler zur Verfügung gestellt.

Insbesondere bei multimorbiden Patienten ist die Anzahl an Daten, die für jeden einzelnen Patienten durch das Patientendaten-Managementsystem erfasst werden müssen, außerordentlich hoch. Aufgrund des erheblichen medizinischen Fortschrittes werden inzwischen jedoch bei vermeintlich einfachen Erkrankungen eine Vielzahl von Daten erfasst, die eine sachgerechte Analyse oft nicht zulassen. Es ist daher nicht untypisch, dass medizinische Entscheidungen anhand einzelner Daten, die als besonders auffallend gelten, getroffen werden. Andere, im Patientendaten-Managementsystem gespeicherte Daten, die einen anderen Befund stützen könnten, werden dabei außer acht gelassen, so dass aufgrund eher willkürlich ausgewählter Daten medizinische Maßnahmen getroffen werden, die sich bei vollständiger Analyse aller Daten als falsch oder unzureichend erweisen könnten.

Überdies ist es ein häufiges Problem, dass tatsächlich nicht alle Daten des Patienten, die erfasst werden sollten, tatsächlich auch erfasst werden. Dennoch ist oft für die verantwortlichen Ärzte und Pfleger nicht zu erkennen, dass Daten fehlen.

Andererseits werden in der Praxis häufig Daten erfasst, die keinen Nutzen für die Diagnose und Therapie einer Erkrankung des Patienten haben. Beispielsweise werden nach starren Schemen Laborwerte angefordert, wobei je nach Wert beispielsweise tägliche oder wöchentliche Bestimmungen vorgenommen werden. Derartige Messungen sind zeit- und kostenaufwendig und können den Patienten zusätzlich belasten.

Ein weiteres schwerwiegendes Problem besteht darin, dass bestimmte Daten zu einem Zeitpunkt in das Patientendaten-Managementsystem gelangen, zu dem sie nicht mehr aktuell ist. Das hat zur Folge, dass den Daten möglicherweise eine Bedeutung beigemessen wird, die ihnen bei richtiger zeitlicher Einordnung nicht zukommen würde. Dies ist insbesondere dann nachteilig, wenn die im Patientendaten-Managementsystem gespeicherten Daten nach vorgegebenen Kriterien analysiert werden und auf Basis dieser Analyse Alarme ausgelöst werden.

Wird ein Alarm ausgelöst, so findet er in der klinischen Praxis häufig nicht die gebotene Aufmerksamkeit, da die Zahl von Alarmierungen beispielsweise in einer intensivmedizinischen Einrichtung derart häufig ist, dass nicht jeder Alarm sofort beachtet werden kann. Zwischen der Alarmierung und der Einleitung der erforderliche medizinischen Maßnahme vergeht daher Zeit, die im schlimmsten Fall den Erfolg der Behandlung ganz oder teilweise beeinträchtigen kann. Überdies ist selbst für einen geschulten Praktiker auch bei einer sofortigen Beachtung des Alarms in den meisten Fällen nicht ohne weiteres klar, welche medizinische Maßnahme zu treffen ist. Die hohe Zahl der angesammelten Daten macht daher eine Gewichtung der Daten erforderlich. Hierbei könnten Klassifikationsverfahren von Nutzen sein, wie sie beispielsweise in EP 1 687 756 A1 beschrieben werden. Auf einem Klassifikationsverfahren kann schließlich ein Entscheidungs-Unterstützungssystem aufbauen, dass den Arzt zu einer sachgerechten Diagnose führen sollte. Solche Entscheidungs-Unterstützungssysteme basieren beispielsweise auf Richtlinien, die von medizinischen Gesellschaften herausgegeben werden, im Falle der Sepsis beispielsweise der Richtlinien der Surviving Sepsis Campaign (SSC).

All diese Faktoren führen insbesondere im intensivmedizinischen Bereich zu einer Fehlerkaskade, die bei der Behandlung eines Patienten ausgelöst werden kann: Die Daten sind unvollständig; die vorhandenen Daten haben eine falsche zeitliche Einordnung; die Alarmierung wird zu spät beachtet, die Daten werden falsch gewichtet, die Diagnose ist daher falsch, oder, sollte sie überhaupt richtig sein, so wird sie spät getroffen.

Dokument WO2010/009717 offenbart ein System das den nächstliegenden Stand der Technik repräsentiert.

Aufgabe der Erfindung ist es, die Nachteile nach dem Stand der Technik zu beseitigen. Es soll insbesondere ein Verfahren zum Überwachen der medizinischen Parameter eines Patienten angegeben werden, dass eine sachgerechte medizinische Behandlung ermöglicht, womit auch eine deutliche Reduzierung der Behandlungszeit eines Patienten verbunden sein kann.

Diese Aufgabe wird durch die Merkmale des Anspruchs 1 gelöst. Zweckmäßige Ausgestaltungen der Erfindungen ergeben sich aus den Merkmalen der Ansprüche 2 bis 5.

Nach Maßgabe der Erfindung ist ein Verfahren gemäß Anspruch 1 vorgesehen.

Der Ausdruck "Zeitpunkt des Ereignisses des Wertes", im Folgenden auch als "Ereignis-Zeitpunkt" bezeichnet, bezieht sich auf den Zeitpunkt des Ereignisses, an dem der Wert eines Parameters bei einem Patienten tatsächlich aufgetreten ist. Dieser Zeitpunkt ist unabhängig davon, wann die Bestimmung des Wertes tatsächlich vorgenommen wird. Dieser Zeitpunkt ist ebenfalls unabhängig davon, wann der Wert tatsächlich in dem System erfasst wird. War eine Probenahme erforderlich, wie dies beispielsweise bei Parametern der Fall ist, die im Labor bestimmt werden müssen, so ist der Zeitpunkt der Probennahme der Ereignis-Zeitpunkt.

Der Ausdruck "Zeitpunkt der Erfassung des Wertes", im Folgenden auch als "Erfassungs-Zeitpunkt" bezeichnet, bezieht sich auf den Zeitpunkt, in dem der Wert in das System gelangt. Jedem Wert kann automatisch der Erfassungs-Zeitpunkt von dem System zugeordnet werden.

Durch die Verknüpfung jedes Wertes mit zwei Zeitstempeln wird sichergestellt, dass in Schritt (c) nur Werte berücksichtigt werden, die tatsächlich den gleichen ersten Zeitstempel aufweisen. Damit ist sichergestellt, dass bei einer Analyse der Daten tatsächlich nur Daten mit dem gleichen Ereignis-Zeitpunkt berücksichtigt werden. Dies ist insbesondere dann von Bedeutung, wenn zwischen dem Ereignis-Zeitpunkt und dem Erfassungs-Zeitpunkt eine erhebliche Zeitdifferenz liegt. Beispielsweise können bei Laborwerten zwischen dem Ereignis-Zeitpunkt und dem Erfassungs-Zeitpunkt drei Tage liegen.

Ein Zeitstempel ist ein Wert in einem vorgegebenen Format, der ein Ereignis einem bestimmten Zeitpunkt zuordnet. Der Zeitstempel kann beispielsweise Tag, Datum, Jahr, Stunde, Minute und Sekunde des Zeitpunktes des Ereignisses kodieren. Zur Vereinfachung des Vergleiches in Schritt (c) kann ein Toleranzwert vorgegeben sein. Dieser Toleranzwert kann beispielsweise 2 s, 5 s, 10 s, 30 s, 1 min, 2 min, 5 min, 10 min, 15 min oder 30 min betragen. Bei dem in Schritt (c) vorgesehenen Vergleich werden dann alle die Werte berücksichtigt, deren erste Zeitstempel sich maximal um den Toleranzwert unterscheiden. Auf diese Weise wird sichergestellt, dass geringfügige zeitliche Unterschiede, beispielsweise weniger als 2 s, zwischen den Werten verschiedener Parameter dazu führen, dass für einen Parameter Werte vorliegen, für einen anderen aber nicht. Alternativ oder zusätzlich kann vorgesehen sein, dass jedem Parameter eine Gültigkeitsdauer zugeordnet ist, beispielsweise 2 s, 5 s, 10 s, 30 s, 1 min, 2 min, 5 min, 10 min, 15 min oder 30 min, 1 h, 2 h, 1 d, 2 d usw. Diese Werte können dann in Schritt (c) verwendet werden, bis, gerechnet vom ersten Zeitstempel an, die Gültigkeitsdauer abgelaufen ist.

Unter den Ausdruck "gleiche erste Zeitstempel" sind also Zeitstempel zu verstehen, die (i) exakt dieselben Zeitstempel sind und/oder die (ii) sich maximal um einen vorgegebenen Toleranzwert unterscheiden und/oder die (iii) innerhalb der vorgegebenen Gültigkeitsdauer liegen.

In der Ausführungsform der Erfindung umfasst Schritt (b) das Auslösen eines Alarmes, wenn für einen Wert ein zweiter Zeitstempel vorliegt, nicht aber ein erster Zeitstempel. Mittels dieser Überprüfung wird sichergestellt, dass alle Werte, die in das System gelangen, bei einer Analyse der Daten nach ihrem Ereignis-Zeitpunkt und nicht nach ihrem Erfassungs-Zeitpunkt berücksichtigt werden.

Erfindungsgemäß sieht Schritt (d) das Auslösen eines Alarms vor, wenn einer der erfassten Werte außerhalb eines vorgegebenen Toleranzbereiches liegt. Dazu sind für jeden Parameter Vergleichswerte und Toleranzen vorgegeben, die in dem System hinterlegt sind. Der Zeitpunkt der Auslösung eines Alarms wird vorzugsweise mit dem Alarm verknüpft.

Nach der Auslösung eines Alarms wird automatisch der Zeitpunkt bestimmt, an dem, gerechnet vom dritten Zeitstempel an, spätestens eine medizinische Maßnahme getroffen werden muss. Die Bestimmung des Zeitpunktes erfolgt auf Basis von Fristen, die in dem System hinterlegt sind. Die Länge der Frist kann vorgegebenen werden, beispielsweise auf Basis von Richtlinien wie denen der Surviving Sepsis Campaign (SSC). Beispielsweise kann vorgegeben werden, dass eine medizinische Maßnahme spätestens nach Ablauf von 10 min, 20 min, 30 min, 45 min, 60 min, 90 min, 120 min getroffen werden muss. Läuft diese Frist ab, ohne dass eine medizinische Maßnahme getroffen worden ist, wird vorzugsweise erneut ein Alarm ausgegeben.

Zur Bestimmung der Länge der Frist kann entweder vorgesehen sein, dass nach jedem Alarm innerhalb einer vorgegebenen Frist eine medizinische Maßnahme zu treffen ist, und zwar unabhängig von dem oder den Parametern, die zur Auslösung des Alarms in Schritt (d) geführt haben. Beispielsweise kann vorgesehen sein, dass nach Auslösung eines Alarmes grundsätzlich innerhalb von 1 h eine medizinische Maßnahme getroffen werden muss. Alternativ kann jedoch vorgesehen sein, die Frist in Abhängigkeit von dem oder den Parametern, die zur Auslösung des Alarms geführt haben, bestimmt wird. Dazu sind in dem System Vorgaben hinterlegt, aus den für jeden Parameter oder jede Gruppe von Parametern spezifische Fristen hinterlegt sind. Liegen beispielsweise die Werte der Atemfrequenz außerhalb des Toleranzbereiches kann eine Frist von 1 h vorgegeben sein, während bei einem abweichenden Wert der Herzfrequenz eine Frist von 20 min vorgegeben sein kann.

Erfindungsgemäß ist ferner die kontinuierliche Ausgabe des in Schritt (f) bestimmten End-Zeitpunktes und/oder des Zeitraumes, der bis zum Erreichen des End-Zeitpunktes verbleibt, und/oder ein Zeitbereich, der kürzer als dieser Zeitraum ist, vorgesehen. Damit wird sichergestellt, dass der Arzt sich zu jedem Zeitpunkt bewusst ist, welcher Zeitraum noch verbleibt, um eine geeignete medizinische Maßnahme zu treffen. Die Ausgabe des End-Zeitpunktes oder des Zeitraums bis zum Erreichen des Endzeitpunktes erfolgt vorzugsweise ständig auf einer Anzeigeeinrichtung des Systems. Alternativ oder zusätzlich kann vorgesehen sein, dass ein Zeitbereich ausgegeben wird, der kürzer als der Zeitraum, der bis zum Erreichen des Endzeitpunktes verbleibt ist.

Vorzugsweise werden in Schritt (g) zusätzlich auch der oder die Werte ausgegeben, die die Auslösung des Alarms in Schritt (d) verursacht haben. Auf diese Weise ist sofort erkennbar, welcher oder welche Werte die Auslösung des Alarms und damit die Bestimmung des End-Zeitpunktes verursacht haben, was eine Entscheidung über eine eventuell zutreffende medizinische Maßnahme weitere vereinfacht.

In einer Ausführungsform der Erfindung kann vorgesehen sein, dass mit der Ausgabe des Alarms in Schritt (d) eine Aufforderung zur manuellen Eingabe von Informationen ausgegeben wird, wobei die Anforderung auf Basis von Richtlinien generiert wird, die in dem System hinterlegt sind und wobei weitere Aufforderungen zur manuellen Eingabe von Informationen auf Basis der Richtlinien ausgegeben werden können, sobald eine manuelle Eingabe der angeforderten Informationen erfolgt. Auf diese Weise kann ein Arzt in Form von Fragen zu einer Entscheidung über die zutreffende medizinische Maßnahme geführt werden, was sich insbesondere in Stresssituationen als nützlich erweist. Die Aufforderungen, d .h der Inhalt der Fragen, werden auf Basis der Richtlinien generiert, wie sie beispielsweise von der Surviving Sepsis Campaign (SSC) vorgegeben werden. Ferner kann vorgesehen sein, dass jede Ausgabe einer Aufforderung mit der Ausgabe des in Schritt (f) bestimmten End-Zeitpunktes und/oder des Zeitraumes, der bis zum Erreichen des End-Zeitpunktes verbleibt, verbunden ist. Um die Beantwortung der Fragen zu erleichtern, kann ferner vorgesehen sein, dass zumindest eine der Aufforderungen zur manuellen Eingabe von Informationen mit der Ausgabe von Werten vorgegebener Parameter verbunden ist, wobei in dem System gespeichert ist, mit welchen Parametern die Aufforderung verbunden ist.

Der in Schritt (g) alternativ oder zusätzlich ausgegebene Zeitbereich beginnt vorzugsweise automatisch mit der manuellen Eingabe von Informationen nach der ersten Ausgabe einer Aufforderung zur manuellen Eingabe von Informationen. Alternativ kann eine gesonderte Aufforderung zum Start des Zeitbereiches ausgegeben werden, so dass der Zeitbereich nur dann beginnt, wenn der Benutzer es wünscht. Der Zeitbereich endet vor dem in Schritt (f) berechneten End-Zeitpunkt. Beispielsweise kann vorgesehen sein, dass der Zeitbereich 10 min, 20 min, 30 min, 45 min, 60 min, 90 min, 120 min beträgt, solange er vor dem End-Zeitpunkt endet. Beträgt die in Schritt (f) vorgegebene Frist beispielsweise 6 h und wurde der Alarm um 9.32 Uhr ausgelöst, so ist der End-Zeitpunkt 15.32 Uhr. Um 9.44 Uhr ist der Zeitraum, der bis zum Erreichen des End-Zeitpunktes verbleibt, 5 h und 38 min. Ist ein Zeitbereich von 1 h vorgegeben und beginnt der Zeitbereich automatisch mit der ersten manuellen Eingabe oder in Reaktion auf eine gesonderte Aufforderung um 9.44 Uhr so endet der Zeitbereich um 10.44 Uhr. Die Länge des Zeitbereiches ist in dem System gespeichert. Damit wird beim Start des Zeitbereiches überprüft, ob das Ende des Zeitbereiches vor dem End-Zeitpunkt liegt. Ist das nicht der Fall, werden der End-Zeitpunkt und/oder der Zeitraum, der bis zum Ereichen des Endzeitpunktes verbleibt, ausgegeben. Liegt das Ende des Zeitbereichs vor dem End-Zeitpunkt, so können ausschließlich das Ende des Zeitbereichs oder die Zeit, die bis zum Ereichen des Endes des Zeitbereiches verbleibt, ausgegeben werden.

Die Schritte (e) bis (g) sind nicht zwingend an die Schritte (a) bis (d) gebunden. Es ist ausreichend, sondern können für jeden Fall des Auslösens eines Alarms angewendet werden. Dies gilt auch für die hier beschriebenen Maßnahmen, die auf die Schritte (e) bis (g) Bezug nehmen.

In der Ausführungsform der Erfindung werden in Schritt (a) die physiologischen Parameter einer ersten Gruppe oder einer zweiten Gruppe zugeordnet, wobei die Parameter der ersten Gruppe kontinuierlich bestimmt wurden, und die Parameter der zweiten Gruppe diskontinuierlich bestimmt wurden. Dabei kann bei Parametern der ersten Gruppe der erste Zeitstempel gleich dem zweiten Zeitstempel gesetzt werden, was insbesondere dann vorteilhaft ist, wenn die Zeitdifferenz zwischen dem ersten und dem zweiten Zeitstempel geringer als 10 min, vorzugsweise geringer als 5 min, besonders bevorzugt geringer als 1 min, ist. Auf diese Weise kann das erfindungsgemäße Verfahren vereinfacht werden. Hingegen sollte bei Parametern der zweiten Gruppe ein Alarm ausgelöst werden, wenn sich der erste Zeitstempel von den zweiten Zeitstempel nicht unterscheidet, da dies für eine Fehleingabe oder Manipulation von Werten oder Ereignissen spricht.

Unter dem Begriff "physiologischer und/oder pathologischer Parameter" ist ein Wert zu verstehen, der (a) den Messwert einer gemessenen physiologischen Kenngröße eines Patienten oder eines Indikatorstoffes angibt (z. B. den Sauerstoffpartialdruck bei einer Blutgasanalyse, Atemfrequenz, Körpertemperatur, CKMB-Konzentration; Keimbelastung); und (b) eine Klassifizierung eines physiologischen Zustandes eines Patienten (z. B. gelbe Färbung der Gesichtshaut; Vorhandensein eines bestimmten Keims; Bestehen einer Resistenz) umfassen kann. In einer Ausführungsform der Erfindung kann vorgesehen sein, dass ein optischer oder/und akustischer Alarm ausgelöst wird, wenn bei der Erfassung von Werten von Parametern ein vorgegebener pathogener Keim und/oder eine Resistenz eines festgestellten Keims gegen ein Arzneimittel festgestellt wird, das dem Patienten verordnet werden soll.

Der Ausdruck "Indikatorstoff" bezieht sich hierin auf Verbindungen oder Elemente, die - je nach ihrer Art - in biologischen Systemen produziert werden oder in biologische Systeme eingebracht werden und deren Vorhandensein oder deren Konzentration (z. B. in einem bestimmten Organ) ein Charakteristikum für einen biologischen Prozess oder einen biologischen Zustand ist. Derartige Verbindungen und Elemente umfassen beispielsweise solche, die von Tumorzellen produziert, durch einen Tumor in anderen Körperzellen induziert und/oder als tumorspezifische Stoffe in ihrer Konzentration durch einen Tumor verändert werden. Derartige Indikatorstoffe sind beispielsweise Makromoleküle, z. B. Proteine, oder Spurenelemente. Derartige Verbindungen und Elemente umfassen weiterhin Bonemarker, die für Knochenabbauprozesse wie Osteoporose charakteristisch sind oder Enzyme, die für die Beurteilung der Funktion von Organen wichtig sind.

Der Begriff "Wert" oder "Messwert" bezieht sich hier auf alle im medizinischen Bereich (a) anfallende Zahlenwerte für einen Parameter (z. B. einen Sauerstoffpartialdruck bei einer Blutgasanalyse in Höhe von 81,2 mmHg, eine Atemfrequenz von 15 Atemzügen pro Minute, eine Körpertemperatur von 36,8 °C, CKMB-Konzentration von 152 ng/ml; Keimbelastung ...) oder (b) auf Klassifizierungsergebnisse eines physiologischen Zustandes eines Patienten (z. B. gelbe Färbung der Gesichtshaut: nein, wobei Klassifizierungen mittels Aussagen wie "Nein" einn Zahlenwert, beispielsweise "0" zugeordnet werden sollte).

Vorzugsweise werden zumindest die Werte eines der physiologischen Parameter, die für das Vorliegen einer Gesundheitsstörung charakteristisch sind, unter Verwendung eines Indikatorstoffes bestimmt.

Dabei sollten physiologische Parameter ausgewählt werden, von denen aufgrund medizinischer Erkenntnisse angenommen wird, dass sie im Zusammenhang mit einer bestimmten Gesundheitsstörung, beispielsweise einer respiratorischen Insuffizienz, stehen. Zur Bestimmung des medizinischen Risikos einer respiratorischen Insuffizienz werden zum Beispiel physiologische Parameter eingesetzt, die mittels einer Blutgasanalyse gewonnen werden. Die mittels der Blutgasanalyse gewonnenen physiologischen Parameter können den pH-Wert des Blutes, den Sauerstoff-Partialdruck des Blutes, den Kohlendioxid-Partialdruck des Blutes und die Sauerstoffsättigung des Blutes umfassen. Weitere physiologische Parameter, die neben den aus der Blutgasanalyse stammenden Werten zur Abschätzung des medizinischen Risikos einer respiratorischen Insuffizienz in dem erfindungsgemäßen Verfahren verwendet können, umfassen die Atemfrequenz (AF) des Patienten sowie das Alter und das Geschlecht des Patienten.

Die Zahl von Parametern, deren Werte in Schritt (a) erfasst werden, sollte mindestens 2 betragen. Werden die Parameter einer ersten und einer zweiten Gruppe zugeordnet, sollte die Zahl von Parametern der ersten Gruppe mindestens 1, vorzugsweise mindestens 2 betragen, während die Zahl von Parametern der zweiten Gruppe, unabhängig davon mindestens 1, vorzugsweise mindestens 2 betragen sollte.

Ein Parameter wird der ersten Gruppe zugeordnet, wenn er kontinuierlich erfasst wurde. Unter kontinuierlichem Erfassen wird das ununterbrochene Erfassen von Werten des Parameters oder das Erfassen von Werten des Parameters in Abständen von 24 h oder weniger verstanden.

Ein Parameter wird der zweiten Gruppe zugeordnet, wenn er diskontinuierlich erfasst wurde. Unter diskontinuierlichem Erfassen wird das einmalige Erfassen von Werten des Parameters; das Erfassen von Werten des Parameters in regelmäßigen oder unregelmäßigen Abständen von mehr als 24 Stunden; oder das vollkommene Fehlen der Erfassung von Werten für diesen Parameter verstanden, wenn sich zu keinem Zeitpunkt ergibt, dass die Erfassung von Werten für diesen Parameter notwendig ist. Dazu umfasst das Verfahren das kontinuierliche Bestimmen der Notwendigkeit von Parametern der zweiten Gruppe auf Basis von zuvor erfassten Werten von Parametern der ersten Gruppe oder auf Basis von zuvor erfassten Werten von Parametern der ersten Gruppe und der zweiten Gruppe.

Ergibt sich, dass ein Wert, der ursprünglich der zweiten Gruppe zugeordnet worden ist, in Abständen von 24 Stunden oder weniger erfasst wurde, so wird dieser Parameter von der zweiten Gruppe in die erste Gruppe eingeordnet. Eine solche Notwendigkeit kann beispielsweise dann vorliegen, wenn ein Parameter, der ursprünglich der zweiten Gruppe zugeordnet worden ist, in zwei oder mehr aufeinander folgenden Abständen von weniger als 24 Stunden bestimmt worden ist. Dazu kann in dem erfindungsgemäßen Verfahren vorgesehen sein, die Abstände zwischen zwei aufeinanderfolgenden Bestimmungen der Parameters, die der zweiten Gruppe zugeordnet sind, zu erfassen und zu überprüfen, ob zwei oder mehr aufeinanderfolgende Abstände zwischen Erfassungen desselben Parameters 24 Stunden oder weniger betragen. Die Anzahl der hierfür erforderlichen aufeinanderfolgenden Abstände, die 24 Stunden oder weniger betragen, sollte eine Ganzzahl größer oder gleich 2 sein.

Wird ein Parameter von der zweiten Gruppe in die erste Gruppe überführt, so kann vorgesehen sein, dass ein Alarm ausgelöst wird und/oder dass die Kategorie in Schritt (e) diesem Parameter, der nun ein Parameter der ersten Gruppe ist, zugeordnet wird.

Der Begriff "medizinische Maßnahme" umfasst jede ärztliche oder pflegerische Maßnahme, die unternommen wird, um den Gesundheitszustand des Patienten zu verbessern, beispielsweise einen oder mehrere der erfassten physiologischen Parameter in einen Bereich zu führen, der bei gesunden Personen typischerweise vorliegt.

Die getroffenen medizinischen Maßnahmen können ebenfalls kontinuierlich erfasst werden, d. h. jede weitere Maßnahme, jede Veränderung einer bestehenden Maßnahme (z. B. der Dosierung eines Medikamentes) und/oder das tatsächliche Ende einer Maßnahme werden erfasst.

In einer weiteren Ausführungsform des erfindungsgemäßen Verfahrens kann ein zusätzlicher Schritt vorgesehen sein, bei dem die Vollständigkeit der Daten überprüft wird. Die Überprüfung der Vollständigkeit der Daten kann kontinuierlich erfolgen. Alternativ wird sie jeweils nach Ablauf einer vorgegebenen Zeitspanne, beispielsweise aller vier, acht, sechzehn und/oder vierundzwanzig Stunden, durchgeführt. Diese Zeitspannen können der Schichtdauer oder einem Arbeitstag des Krankenhauses entsprechen.

Die Daten werden dann als vollständig angesehen, wenn sie den medizinischen Vorgaben, insbesondere den getroffenen medizinischen Maßnahmen sowie den physiologischen Parametern und sonstigen Informationen, die gemäß den Vorgaben des Krankenhauses im allgemeinen und den Vorgaben des Arztes im besonderen zu erfassen sind, entsprechen.

Die Daten, die gemäß den medizinischen Vorgaben zu erfassen sind, werden im folgenden auch als "erwartete Daten" bezeichnet, da deren Erfassung gemäß den medizinischen Vorgaben erwartet wird. Einzelne erwartete Daten werden als "erwartete Angabe" bezeichnet.

Um die Vollständigkeit der Daten zu überprüfen, werden die tatsächlich erfassten Daten mit den Daten verglichen, die gemäß den medizinischen Vorgaben zu erfassen sind. Die erfassten Daten werden dabei den erwarteten Daten zugeordnet. Wird beispielsweise die Erfassung des Sauerstoffpartialdruckes erwartet, so wird der tatsächlich gemessene und erfasste Wert des Sauerstoffpartialdruckes dem erwarteten Wert zugeordnet. Jede erwartete Angabe kann mit einer oder mehreren Zeitangaben verknüpft sein, aus der sich ergibt, wann ein Wert für die erwartete Angabe erwartet wird.

In einem Beispiel ist ferner ein System zur Überwachung des medizinischen Zustandes einer Vielzahl von Patienten gemäß dem Verfahren der vorliegenden Erfindung vorgesehen. Das System umfasst einen Prozessor, einen Speicher, eine Eingabeeinrichtung und eine Anzeigeeinrichtung, wobei
- die Eingabeeinrichtung dem Anwender die manuelle Eingabe von Informationen für jeden Patienten ermöglicht;
- in dem Speicher für jeden dieser Patienten die erfassten Werte von Parametern sowie deren erste und zweite Zeitstempel gespeichert sind;
- der Prozessor den kontinuierlichen Vergleich von Werten unterschiedlicher Parameter, die den gleichen ersten Zeitstempel aufweisen mit Vergleichswerten, die in dem System hinterlegt sind; das Auslösen eines Alarms, wenn der Vergleich ergibt, dass zumindest einer der Werte außerhalb eines vorgegebenen Toleranzbereiches in Bezug auf den Vergleichswert liegt; das Verknüpfen des Alarms mit einem dritten Zeitstempel, der den Zeitpunkt der Auslösung des Alarms wiedergibt; und die Bestimmung des Zeitpunktes (End-Zeitpunkt), gerechnet vom dritten Zeitstempel an, an dem spätestens eine medizinische Maßnahme getroffen werden muss, wobei die Bestimmung des Zeitpunktes auf Basis von vorgegebenen Fristen beruht, die in dem System hinterlegt sind; und
- die Anzeigeeinrichtung kontinuierlich den in Schritt (f) bestimmten End-Zeitpunkt und/oder den Zeitraum, der bis zum Erreichen des End-Zeitpunktes verbleibt, oder den Zeitbereich ausgibt.

Ferner können in dem Speicher Datenbanken für die Richtlinien, die Vergleichswerte und deren Toleranzbereiche hinterlegt sein.

Das System kann ferner eine Einrichtung zum Überprüfen der Vollständigkeit der Daten umfassen.

Das System kann ein computerimplementiertes System sein, insbesondere ein computerimplementiertes Patientendaten-Management- und/oder Entscheidungsunterstützungssystem.

Nachfolgend wird die Erfindung anhand von Zeichnungen näher erläutet. Dabei zeigen
- Fig. 1: eine schematische Darstellung einer Ausführungsform des erfindungsgemäßen Verfahrens;
- Fig. 2a-e: schematische Darstellungen von Bildschirmbildern der erfindungsgemäßen Anzeigeeinrichtung; und
- Fig. 3: eine schematische Darstellung eines Bildschirmbildes der erfindungsgemäßen Anzeigeeinrichtung.

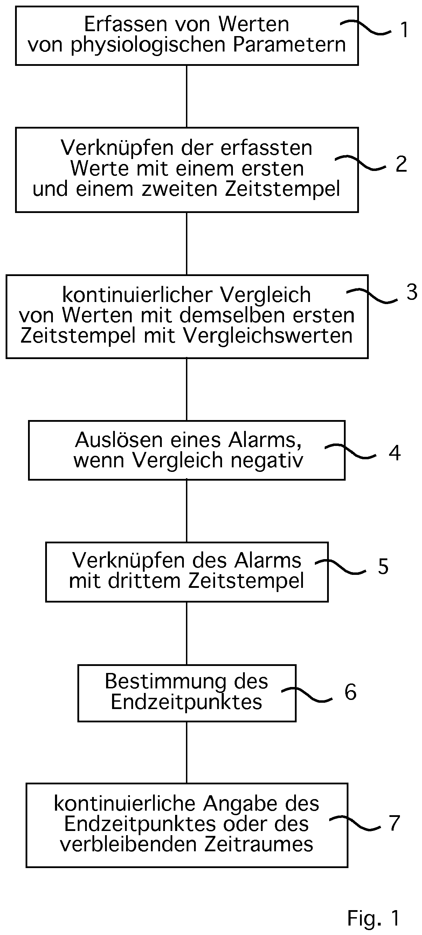

Der Umfang der Erfindung ist durch die Ansprüche definiert. Die folgenden Ausführungsformen definieren nur Beispiele, wovon nur diejenigen, die in den Umfang der Ansprüche fallen, Teil der Erfindung sind. Bei dem in Fig. 1 gezeigten Ausführungsbeispiel werden zunächst kontinuierlich oder diskontinuierlich Werten von physiologischen Parametern des Patienten erfasst, von denen zumindest ein Teil für das Vorliegen einer Gesundheitsstörung charakteristisch ist 1. Die erfassten Werte werden mit einem ersten Zeitstempel und einem zweiten Zeitstempel verknüpft, wobei der erste Zeitstempel den Zeitpunkt des Ereignisses des Wertes und der zweite Zeitstempel den Zeitpunkt der Erfassung des Wertes wiedergibt 2. Anschließend werden kontinuierlich die Werte aller Parameter, die den gleichen ersten Zeitstempel aufweisen, mit Vergleichswerten, die in dem System hinterlegt sind, verglichen 3. Ein gleicher Zeitstempel eines Wertes eines ersten Parameters kann im Verhältnis zu dem Wert eines zweiten (oder beliebigen anderen) Parameters vorliegen, wenn (i) der Wert des ersten Parameters und der Wert des zweiten Parameters exakt dieselben Zeitstempel aufweisen oder (ii) wenn sich der Wert des ersten Parameters von dem Wert des zweiten Parameters maximal um einen vorgegebenen Toleranzwert unterscheidet oder wenn (iii) der Wert des ersten Parameters innerhalb der vorgegebenen Gültigkeitsdauer, gerechnet von seinem ersten Zeitstempel an, liegt, und der erste Zeitstempel des zweiten Parameters ebenfalls innerhalb der Gültigkeitsdauer des Wertes des ersten Parameters liegt oder sich der erste Zeitstempel des zweiten Parameters nur um den vorgegebenen Toleranzwert von der Gültigkeitsdauer des Wertes des ersten Parameters unterscheidet oder wenn (iv) der Wert des zweiten Parameters innerhalb der vorgegebenen Gültigkeitsdauer, gerechnet von seinem ersten Zeitstempel an, liegt, und der erste Zeitstempel des ersten Parameters ebenfalls innerhalb der Gültigkeitsdauer des Wertes des zweiten Parameters liegt oder sich der erste Zeitstempel des ersten Parameters nur um den vorgegebenen Toleranzwert von der Gültigkeitsdauer des Wertes des zweiten Parameters unterscheidet.

Ergibt der Vergleich in Schritt (c), dass zumindest einer der Werte außerhalb eines vorgegebenen Toleranzbereiches in Bezug auf den Vergleichswert liegt, so wird ein Alarm ausgelöst 4. Der Alarm wird dabei mit einem dritten Zeitstempel verknüpft, der den Zeitpunkt der Auslösung des Alarms wiedergibt 5. Anschließend wird der Zeitpunkt (End-Zeitpunkt), gerechnet vom dritten Zeitstempel an, bestimmt, an dem spätestens eine medizinische Maßnahme getroffen werden muss 6. Die Bestimmung des Zeitpunktes wird auf Basis von vorgegebenen Fristen vorgenommen, die in dem System hinterlegt sind. Der so ermittelte End-Zeitpunkt und/oder der Zeitraum, der bis zum Erreichen des End-Zeitpunktes verbleibt, werden dann auf der Anzeigeeinrichtung des Systems ausgegeben.

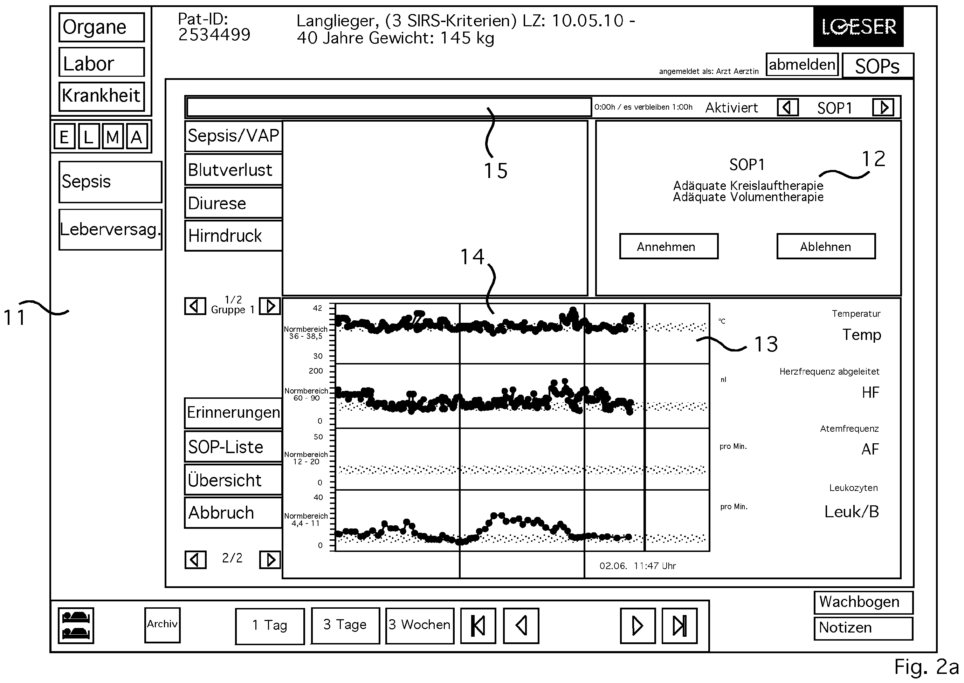

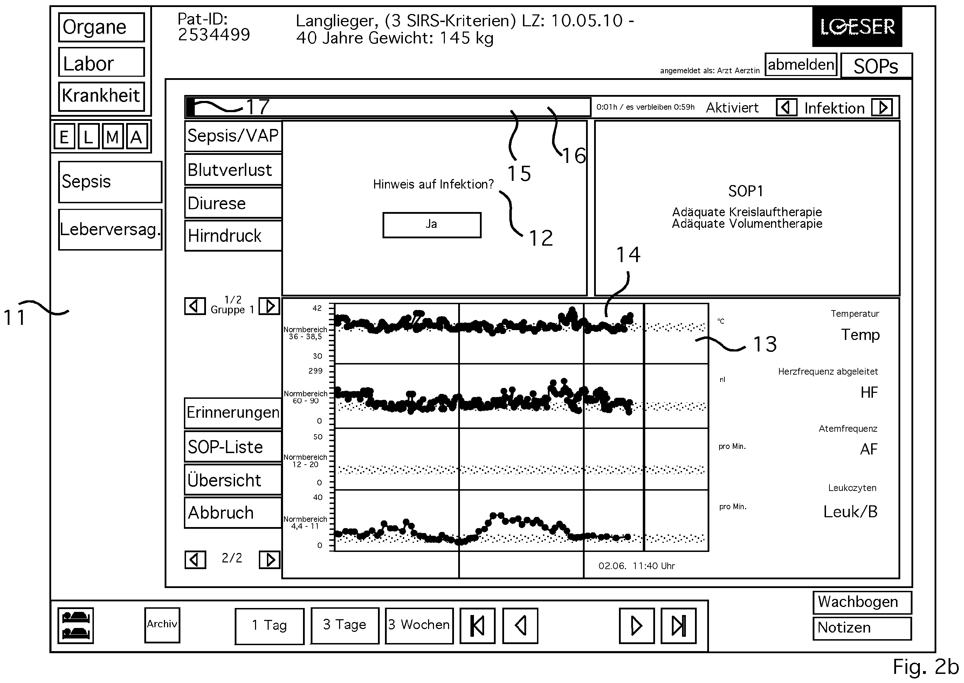

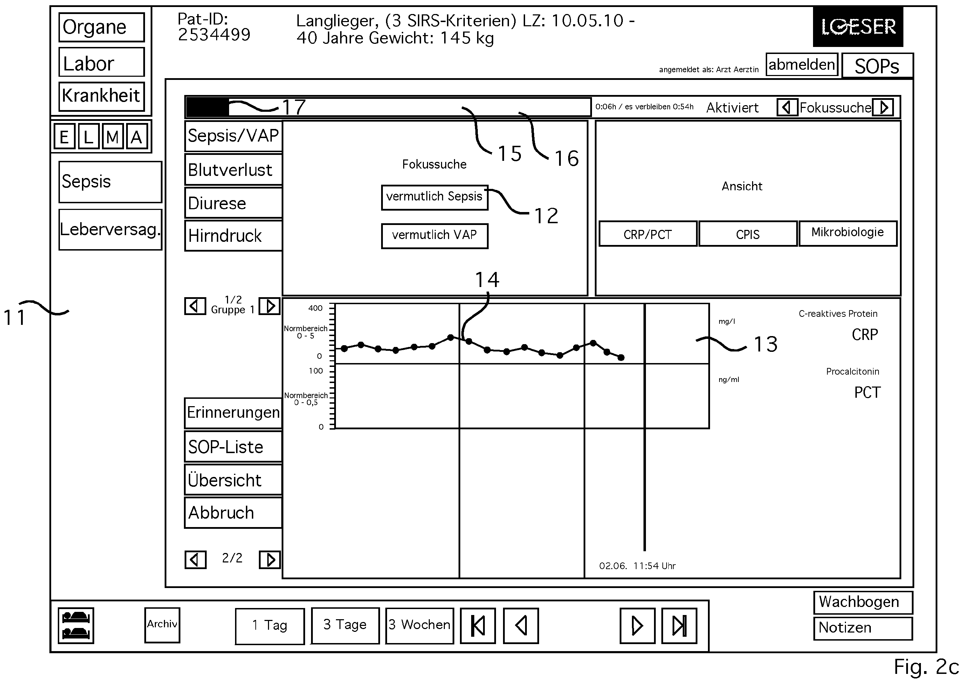

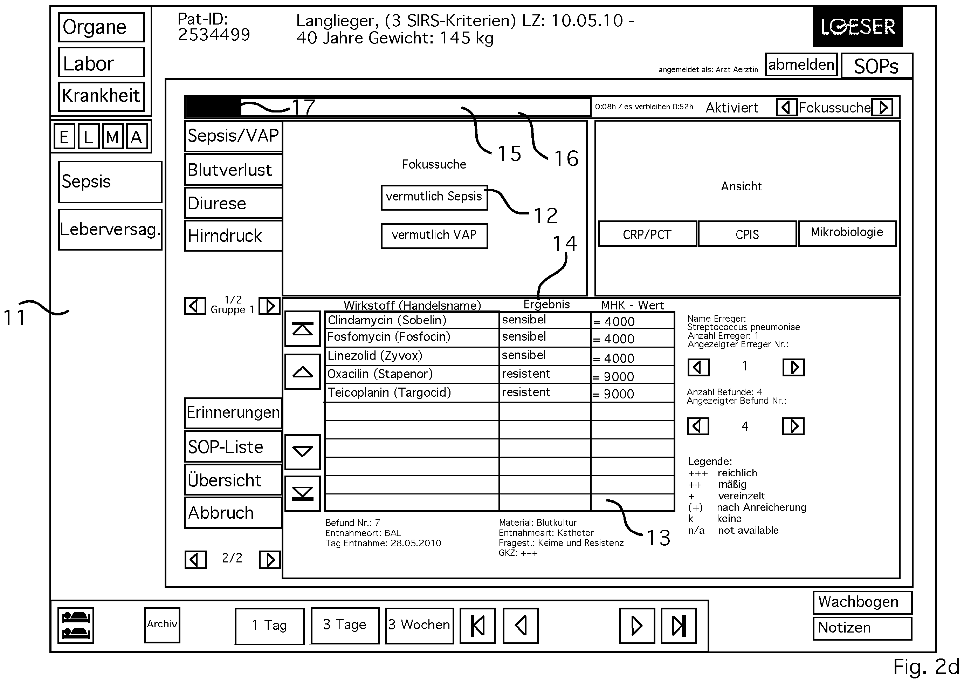

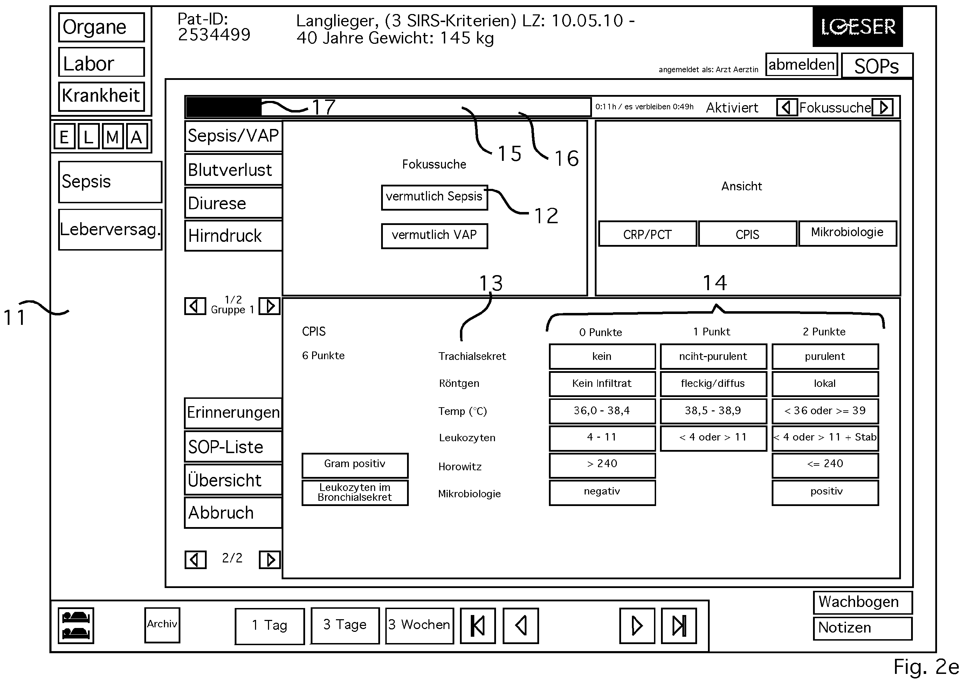

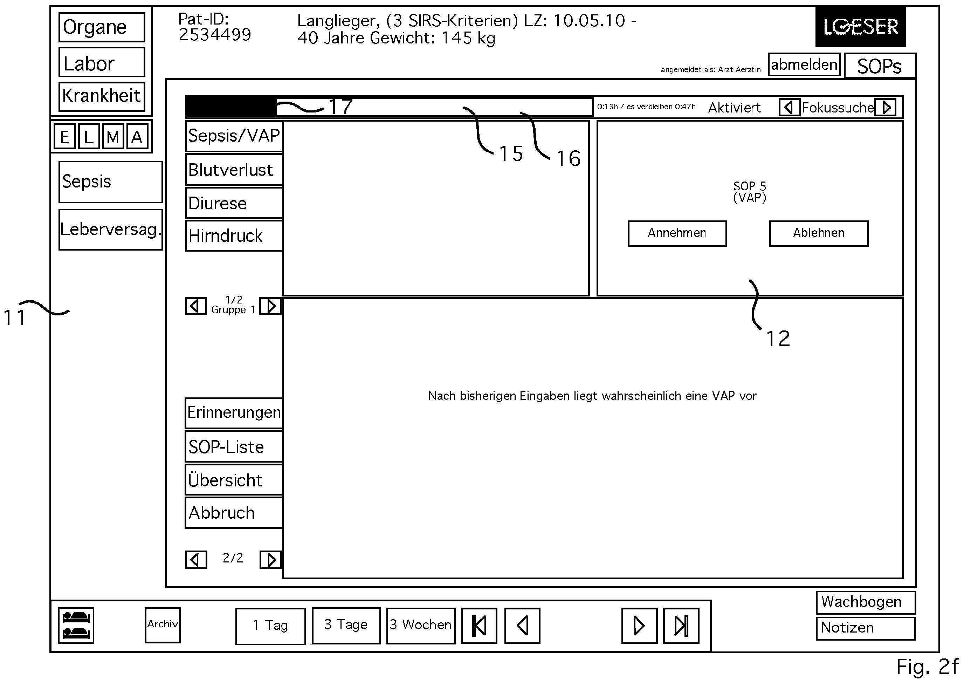

Gemeinsam mit der Ausgabe des Alarms können nacheinander mehrere Aufforderungen zur manuellen Eingabe von Informationen auf der Anzeigeeinrichtung ausgegeben werden, die auf Basis von Richtlinien generiert worden sind, die in dem System hinterlegt sind. In Fig. 2a-e sind Bildschirmdarstellungen 11 der Anzeigeinrichtung gezeigt, auf denen derartige Aufforderungen 12 gemeinsam mit Werten 14 von Parametern 13 dargestellt werden, die dem Benutzer die Eingabe der angeforderten Informationen erleichtern sollen. Außerdem wird der Zeitraum, der bis zum Erreichen des End-Zeitpunktes verbleibt, in Form eines Statusbalkens 15 angezeigt (Fig. 2a). In den folgenden Figuren 2b bis 2f ist der verbleibende Zeitraum 16 jeweils farblich von dem bereits verstrichenen Zeitraum 17 seit Auslösung des Alarms getrennt dargestellt. Gibt der Benutzer die angeforderten Informationen, wie in der Reihenfolge der Figuren dargestellt, ein, so generiert das Entscheidungsunterstützungssystem schließlich einen Vorschlag, der den Benutzer bei der Findung einer Diagnose und der Auswahl einer geeigneten medizinischen Maßnahme unterstützen kann (Fig. 2f).

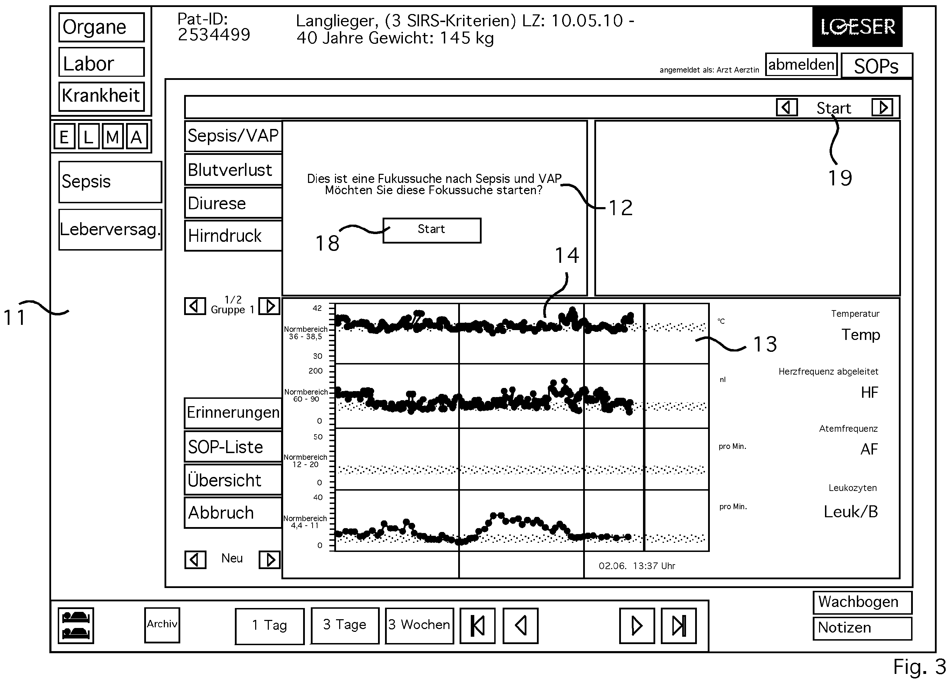

In Fig. 3 ist ein Bildschirmbild gezeigt, mit dem eine erste Aufforderung zur manuellen Eingabe von Daten ausgegeben wird. Der Beginn des Zeitbereiches kann dabei automatisch mit der manuellen Eingabe von Informationen durch Betätigen der Software-Taste 18 oder alternativ durch Betätigen der Software-Taste 19 gestartet werden, wenn kein automatischer Start vorgesehen ist. Werden nun das Ende der Zeitbereiches und die Zeit bis zum Ende des Zeitbereiches auf der Anzeigevorrichtung ausgegeben, so entsprechen die dem Benutzer dann angezeigten Bildschirmbilder den in Fig. 2a bis 2f gezeigten Bildschirmbilder mit der Ausnahme, dass anstelle des End-Zeitpunktes und des Zeitraumes, der bis zum Erreichen des End-Zeitpunktes verbleibt, das Endes des Ende der Zeitbereiches und die Zeit, die bis zum Ende des Zeitbereiches verbleibt, dargestellt sind.

## Patentansprüche

1. Verfahren zur computergestützten Überwachung der medizinischen Parameter eines Patienten mittels eines Patientendaten-Management- und/oder Entscheidungsunterstützungssystems, umfassend die Schritte
(a) das Zuordnen von physiologischen Parametern des Patienten zu einer ersten Gruppe oder einer zweiten Gruppe, wobei die physiologischen Parameter erfasst wurden (1), wobei zumindest ein Teil der physiologischen Parameter für das Vorliegen einer Gesundheitsstörung charakteristisch ist und wobei die physiologischen Parameter
- Messwerte einer gemessenen physiologischen Kenngröße des Patienten oder eines Indikatorstoffes oder
- Klassifizierungen eines physiologischen Zustandes eines Patienten
sind und wobei die Parameter der ersten Gruppe kontinuierlich bestimmt wurden und die Parameter der zweiten Gruppe diskontinuierlich bestimmt wurden und wobei
die Notwendigkeit von Parametern der zweiten Gruppe auf Basis von zuvor erfassten Werten von Parametern der ersten Gruppe oder auf Basis von zuvor erfassten Werten von Parametern der ersten Gruppe und der zweiten Gruppe kontinuierlich bestimmt wird, wobei ein Parameter, der ursprünglich der zweiten Gruppe zugeordnet worden ist und in Abständen von 24 Stunden oder weniger erfasst wurde, von der zweiten Gruppe in die erste Gruppe eingeordnet wird;
(b) das Verknüpfen (2) von erfassten Werten mit einem ersten Zeitstempel und einem zweiten Zeitstempel, wobei der erste Zeitstempel den Zeitpunkt des Ereignisses des Wertes und der zweite Zeitstempel den Zeitpunkt der Erfassung des Wertes wiedergibt, wobei bei Parametern der ersten Gruppe der erste Zeitstempel gleich dem zweiten Zeitstempel gesetzt wird, wenn die Zeitdifferenz zwischen dem ersten und zweiten Zeitstempel geringer als 10 min ist, und bei Parametern der zweiten Gruppe ein Alarm ausgelöst wird, wenn sich der erste Zeitstempel von dem zweiten Zeitstempel nicht unterscheidet, und ein Alarmausgelöst wird, wenn für einen Wert ein zweiter Zeitstempel vorliegt, nicht aber ein erster Zeitstempel;
(c) den kontinuierlichen Vergleich (3) von Werten unterschiedlicher Parameter, die den gleichen ersten Zeitstempel aufweisen, mit Vergleichswerten, die in dem System hinterlegt sind, wobei gleiche erste Zeitstempel erste Zeitstempel sind, die sich maximal um einen Toleranzwert unterscheiden;
(d) das Auslösen (4) eines Alarms, wenn der Vergleich ergibt, dass zumindest einer der Werte außerhalb eines vorgegebenen Toleranzbereiches in Bezug auf den Vergleichswert liegt;
(e) das Verknüpfen (5) des Alarms mit einem dritten Zeitstempel, der den Zeitpunkt der Auslösung des Alarms wiedergibt;
(f) die Bestimmung (6) des Zeitpunktes (End-Zeitpunkt), gerechnet vom dritten Zeitstempel an, an dem spätestens eine medizinische Maßnahme getroffen werden muss, wobei die Bestimmung des Zeitpunktes auf Basis von vorgegebenen Fristen vorgenommen wird, die in dem System hinterlegt sind; und
(g) die kontinuierliche Ausgabe (7) (i) des in Schritt (f) bestimmten End-Zeitpunktes und/oder (ii) des Zeitraumes, der bis zum Erreichen des End-Zeitpunktes verbleibt, und/oder (iii) eines Zeitbereiches, der kürzer als der Zeitraum ist, der bis zum Erreichen des End-Zeitpunktes verbleibt.

2. Verfahren nach Anspruch 1, **dadurch gekennzeichnet, dass** in Schritt (g) zusätzlich der oder die Werte ausgegeben werden, die die Auslösung des Alarms in Schritt (d) verursacht haben.

3. Verfahren nach Anspruch 1 oder Anspruch 2, **dadurch gekennzeichnet, dass** mit der Ausgabe des Alarms in Schritt (d) eine Aufforderung zur manuellen Eingabe von Informationen ausgegeben wird, wobei die Anforderung auf Basis von Richtlinien generiert wird, die in dem System hinterlegt sind, und wobei weitere Aufforderungen zur manuellen Eingabe von Informationen auf Basis der Richtlinien ausgegeben werden können, sobald eine manuelle Eingabe der angeforderten Informationen erfolgt.

4. Verfahren nach Anspruch 3, **dadurch gekennzeichnet, dass** jede Ausgabe einer Aufforderung mit der Ausgabe des in Schritt (f) bestimmten End-Zeitpunktes und/oder des Zeitraumes, der bis zum Erreichen des End-Zeitpunktes verbleibt, verbunden ist.

5. Verfahren nach Anspruch 3 oder Anspruch 4, **dadurch gekennzeichnet, dass** zumindest eine der Aufforderungen zur manuellen Eingabe von Informationen mit der Ausgabe von Werten vorgegebener Parameter verbunden ist, wobei in dem System gespeichert ist, mit welchen Parametern die Aufforderung verbunden ist.

## Claims

1. A method for the computer-assisted monitoring of the medical parameters of a patient by means of a patient data management and/or decision support system comprising the steps of
(a) assigning physiological parameters of the patient to a first group or a second group, wherein the physiological parameters have been acquired (1), wherein at least a part of the physiological parameters is characteristic for the presence of a disturbance of health and wherein the physiological parameters are
- measured values of a measured physiological index of the patient or an indicator substance or
- classifications of a physiological condition of a patient
and wherein the parameters of the first group were continuously determined, and the parameters of the second group were discontinuously determined and wherein
the necessity of parameters of the second group is continuously determined on the basis of previously acquired values of parameters of the first group or on the basis of previously acquired values of parameters of the first group and the second group, wherein a parameter that originally has been assigned to the second group and was acquired in intervals of 24 hours or less is sorted from the second group into the first group;
(b) linking (2) acquired values to a first time stamp and a second time stamp, wherein the first time stamp depicts the moment of the incident of the value and the second time stamp depicts the moment of the acquisition of the value, wherein with parameters of the first group the first time stamp is set equal to the second time stamp if the time difference between the first and second time stamp is lower than 10 min, and with parameters of the second group an alert is triggered if the first time stamp is not different from the second time stamp, and an alert is triggered if there is a second time stamp for one value but not a first time stamp;
(c) continuously comparing (3) values of different parameters having the same first time stamp with comparative values being stored in the system, wherein same first time stamps are first time stamps differing by at most a tolerance value;
(d) triggering (4) an alert if the comparison shows that at least one of the values lies outside of a given tolerance range with respect to the comparative value;
(e) linking (5) the alert to a third time stamp depicting the time of the triggering of the alert;
(f) determining (6) the time (end time) calculated beginning from the third time stamp at which a medical measure must be taken at latest, wherein the time is determined based on given terms stored in the system; and
(g) continuously outputting (7) (i) the end time determined in step (f) and/or (ii) the period remaining to reach the end time and/or (iii) a time range being shorter than the period remaining to reach the end time.

2. The method according to claim 1, **characterized in that** in step (g) additionally the value(s) are outputted that have caused the triggering of the alert in step (d).

3. The method according to claim 1 or claim 2, **characterized in that** with the output of the alert in step (d) a request to manually input information is output, wherein the request is generated on the basis of guidelines stored in the system, and wherein further requests to manually input information can be output on the basis of the guidelines as soon as the requested information are manually input.

4. The method according to claim 3, **characterized in that** each output of a request is associated with the output of the end time determined in step (f) and/or of the period remaining to reach the end time.

5. The method according to claim 3 or claim 4, **characterized in that** at least one of the requests to manually input information is associated with the output of values of given parameters, wherein it is stored in the system with which parameters the request is associated.

## Revendications

1. Procédé de surveillance assistée par ordinateur des paramètres médicaux d'un patient au moyen d'un système de gestion de données de patient et/ou d'aide à la décision, comprenant les étapes
(a) l'attribution des paramètres physiologiques du patient à un premier groupe ou un deuxième groupe, les paramètres physiologiques ayant été saisis (1), au moins certains des paramètres physiologiques étant caractéristiques de la présence d'un trouble de santé et les paramètres physiologiques étant
- les valeurs mesurées d'un paramètre physiologique mesuré du patient ou d'une substance indicatrice, ou
- des classifications d'un état physiologique d'un patient
les paramètres du premier groupe étant déterminés en continu et les paramètres du deuxième groupe étant déterminés de manière discontinue et
la nécessité de déterminer des valeurs de paramètres du deuxième groupe, sur la base de valeurs enregistrées au préalable des paramètres du premier groupe ou sur la base de valeurs enregistrées au préalable des paramètres du premier groupe et du second groupe déterminés en continu, un paramètre devant être initialement affecté au deuxième groupe et devant être enregistré dans des délais de 24 heures ou moins, classé du deuxième groupe dans le premier groupe;
(b) l'association (2) de valeurs enregistrées avec un premier horodatage et un second horodatage, le premier horodatage représentant l'heure de l'événement de la valeur et le second horodatage représentant l'heure de l'enregistrement de la valeur, le premier horodatage étant égal au second pour les paramètres du premier groupe si la différence temporelle entre le premier et le deuxième horodatage est inférieure à 10 min., et une alarme se déclenchant avec les paramètres du deuxième groupe si le premier horodatage ne diffère pas du deuxième, et une alarme se déclenchant si un second horodatage est présent pour une valeur en l'absence d'un premier horodatage;
(c) la comparaison continue (3) de valeurs de différents paramètres présentant le même premier horodatage avec des valeurs de comparaison enregistrées dans le système, les premiers horodatages étant différents des premiers horodatages d'une valeur de tolérance au maximum;
(d) le déclenchement (4) d'une alarme lorsque la comparaison montre qu'au moins une des valeurs se situe en dehors d'une plage de tolérance indiquée par rapport à la valeur de comparaison;
(e) l'association (5) de l'alarme avec un troisième horodatage qui reproduit le moment du déclenchement de l'alarme;
(f) la détermination (6) du moment (terme), à compter du troisième horodatage, auquel une mesure médicale doit être prise au plus tard, la détermination du moment étant déterminé sur la base des délais fixés, enregistrés dans le système; et
(g) l'affichage continu (7) (i) du terme déterminé dans l'étape (f) et /ou (ii) de la période, qui reste jusqu'à l'atteinte du terme, et /ou (iii) d'une plage temporelle plus courte que la période, qui reste jusqu'à l'atteinte du terme.

2. Procédé selon la revendication 1, **caractérisé en ce que** dans l'étape (g), la ou les valeurs qui ont provoqué le déclenchement de l'alarme dans l'étape (d) sont également émises.

3. Procédé selon la revendication 1 ou la revendication 2, **caractérisé en ce que** lors de l'affichage de l'alarme dans l'étape (d), un ordre de saisie manuelle des informations s'affiche, la demande étant générée sur la base de directives enregistrées dans le système et d'autres ordres de saisies manuelles d'informations sur la base des directives pouvant être émis dès qu'une saisie manuelle des informations demandées a eu lieu.

4. Procédé selon la revendication 3, **caractérisé en ce que** chaque affichage d'un ordre est associé à l'affichage du terme déterminé dans l'étape (f) et /ou de la période, qui reste jusqu'à l'atteinte du terme.

5. Procédé selon la revendication 3 ou la revendication 4, **caractérisé en ce qu'**au moins un des ordres de saisie manuelle d'informations est associé à l'affichage de valeurs de paramètres prédéterminés, l'association de l'ordre et des paramètres concernés étant enregistrée dans le système.
